# EUROPEAN PATENT SPECIFICATION

(11) **EP 0 248 416 B1**
(45) Date of publication and mention of the grant of the patent: **19.05.1993**
(21) Application number: 87108031.3
(22) Date of filing: 03.06.1987
(51) Int. Cl.: C07K 5/06

(54) **Method for recovering alpha-L-aspartyl-L-phenylalanine methyl ester**
Methode für Rückgewinnung von Methylester von alpha-L-Aspartyl-L-Phenylalanin
Méthode de récupération d'alpha-aspartyl-L-phénylalanine (ester méthylique)

(30) Priority: 03.06.1986 JP 128405/86
(43) Date of publication of application: 09.12.1987
(73) Proprietor: AJINOMOTO CO., INC., Tokyo 104 (JP)
(72) Inventor: Sugiyama, Katsumi Tokai Plant, Yokkaichi-shi Mie-ken (JP); Yamashita, Takashi Tokai Plant, Yokkaichi-shi Mie-ken (JP); Yukawa, Toshihide Central Research Laboratories, Kawasaki-shi Kanagawa-ken (JP)
(74) Representative: Strehl Schübel-Hopf Groening & Partner

(56) References cited:
- EP-A- 0 092 933
- CHEMICAL ABSTRACTS, vol. 108, no. 7, 15th November 1988, page 780, abstract no. 56620t, Columbus, Ohio, US; & JP-A-62 153 298 (TOYO SODA MFG. CO., LTD) 08-07-1987

## Description

The present invention relates to a method for effectively recovering α-L-aspartyl-L-phenylalanine methyl ester (hereafter simply referred to as α-APM) from a solution mixture containing the methyl ester at the L-aspartic acid β-carboxyl residue of α-L-aspartyl-L-phenylalanine methyl ester (hereafter simply referred to as α-A(M)PM), the demethylated esters thereof (α-APM, α-A(M)PM and α-L-aspartyl-L-phenylalanine (hereafter simply referred to as α-AP)) and an inorganic chloride.

α-APM is a substance whose utility as a low calorie sweetener of the peptide type has recently attracted particular attention.

α-APM has the property in an aqueous solution to be relatively stable at temperatures lower than normal temperature but to readily undergo demethyl-esterification or intramolecular removal of methanol to cyclize and form α-AP or intramolecularly cyclized α-L-aspartyl-L-phenylalanine (hereafter simply referred to as DKP) under severe conditions at high temperatures or in an acidic region, because α-APM is a methyl ester compound of a peptide having 2 amino acids.

Upon isolation and purification of α-APM from the reaction solution in an industrial scale, such severe conditions as high temperatures or acidic conditions are often adopted to enhance the efficiency; in this case, by-production of α-AP or DKP is unavoidable. In addition, α-APM or α-AP is methyl-esterified with by-produced methanol, whereby α-A(M)PM or α-A(M)P is also formed. Upon isolation and purification of α-APM from the reaction solution, the formation of α-A(M)PM or α-A(M)P does not only occur in an aqueous solution but in many cases occurs in a solution mixture of methanol and water; in this case, the formation of α-A(M)PM or α-A(M)P is more accelerated. Therefore, a part of α-APM changes to α-AP, DKP, α-A(M)PM or α-A(M)P during the isolation and purification of α-APM and is a cause for seriously reducing the yield of α-APM. Further, these large quantities of products are contained in the mother liquor from which α-APM is separated.

On the other hand, processes for synthesis of α-APM include a process which comprises reacting N-protected-L-aspartic anhydride and L-phenylalanine methyl ester in an organic solvent and then cleaving off the protective groups in a conventional manner (U.S. Patent 3,786,039), a process which comprises directly reacting strong acid addition salts of L-aspartic anhydride and L-phenylalanine methyl ester (Published Examined Japanese Patent Application No. 14,217/73), etc. However, in any of these processes, the reaction solution is obtained in such a state as containing, in addition to α-APM, impurities such as the by-produced β-form, the unreacted L-phenylalanine and L-aspartic acid used as raw materials or by-products thereof.

Therefore, a method for efficiently separating α-APM of high purity is extremely important for the industrial production of α-APM. As a notable method, there is known a method which comprises contacting impure α-APM with a hydrohalic acid in an aqueous solvent to precipitate the hydrohalic acid salt of α-APM, subjecting the salt to solid-liquid separation and neutralizing with an alkali to give α-APM (U.S. Patent 3,798,207).

In such a case, the mother liquor remained after separating α-APM contains large quantities of salts, generally inorganic chlorides, because hydrochloric acid is used as the hydrohalic acid from an economical standpoint.

Accordingly, the mother liquor exhausted during the isolation and purification of α-APM upon production of α-APM in an industrial scale contains, in addition to α-APM, α-AP formed by conversion of α-APM, DKP, α-A(M)PM, α-A(M)P and large amounts of inorganic chlorides, in admixture. The inorganic chlorides vary depending on the alkali provided for the neutralization of the α-APM hydrochloride but are generally NaCℓ, NH₄Cℓ, KCℓ, etc. Recovery of α-APM from such a mother liquor is extremely advantageous in improving the productivity in the production of α-APM in an industrial scale because such contributes to great improvement in the yield at the isolation and purification step of α-APM and to reduction in production costs of α-APM. However, it was difficult to recover α-APM from the mother liquor economically advantageously due to the following problems.

The first problem is as follows: In order to improve the yield of the product, there is performed a method which comprises recycling the mother liquor, from which the product was once separated, to treating steps, for purposes of enhancing the concentration of the product in the mother liquor by evaporation, concentration, etc. and, again precipitating and separating the product from the mother liquor, repeating such procedures several times. If large quantities of salts are present in the mother liquor, however, the concentration of the salts becomes high and the salts are precipitated as the procedures are repeated; in this case, it is difficult to recover the product in a high yield and recycle the mother liquor. Particularly in the case that it is advantageous to recover the product from the hydrochloric acid solution as for α-APM, the solubility of the inorganic chlorides decreases due to the presence of hydrochloric acid in the system so that it becomes more difficult to recover the product.

Therefore, in case that salts such as inorganic chlorides, etc. are present in large quantities, it is necessary that desalting be previously conducted in any means.

For desalting, there are hitherto known (1) a method using ion exchange resin, (2) a method which comprises adsorbing the product to an adsorbent such as a synthetic adsorbent, activated charcoal, etc., breaking the salts present through a non-adsorbed solution and then eluting with a polar solvent such as methanol, etc., (3) a method utilizing electric dialysis, (4) a method using a ultrafiltration membrane, etc. These methods involve the defects that large quantities of secondary raw materials are consumed for elution and regeneration of ion exchange resins or adsorbents or complicated operations increase the costs for devices ((1) and (2)). In the case that the product has an electrolytic property, desalting is performed and at the same time, the product is dialyzed to decrease the yield ((3)), the method using an ultrafiltration membrane applies generally to the product having a molecular weight of several thousands or more and in the case of the product having a lower molecular weight, the product is also permeated through the membrane so that the yield of the product is markedly reduced, etc. In addition, the desalted solution thus obtained becomes dilute so that much energy is required to concentrate the solution for purposes of recovering the product from the desalted solution. Therefore, the costs for recovery increase which is disadvantageous also from the economic aspect.

A second problem is that it is extremely difficult to crystallize α-APM directly from the impure system where α-A(M)PM, α-A(M)P, α-AP, DKP, etc. are present in large quantities, because precipitation of α-APM crystals is inhibited by these impurities.

Accordingly, there has been heretofore adopted a method which comprises contacting such a solution with a strong acid such as HCℓ, H₂SO₄, etc. at high temperatures thereby to hydrolyze up to the constituent amino acids, fractionally crystallizing L-Phe and L-Asp, recovering each of them independently and then reusing them as raw materials. According to this method, treatment is required under such severe conditions as strong acids and high temperatures so that costs for devices increase to ensure safe operation and resistance to corrosion. In addition, the method involves that acids and alkalis are consumed in large quantities.

In order to overcome these problems, the present inventors have made extensive investigations and as a result, they have found that by desalting and concentrating a specific solution, in which the ratio of the α-A(M)PM content to the sum of α-APM, α-A(M)P and α-AP is not greater than 1.0 in a weight ratio and the ratio of the inorganic chloride content to the sum of them is not less than 1.0 in a weight ratio, with a reverse osmosis membrane having a NaCℓ inhibition rate of 30 to 80% until the ratio of the inorganic chloride content becomes 0.3 to 1.0, then bringing the concentrate into contact with a methanol-hydrochloric acid aqueous solution and precipitating crystals of the α-APM hydrochloride, α-APM can be efficiently recovered from the solution in a high recovery yield and have come to accomplish the present invention.

The feature of the present invention lies in desalting the solution using a reverse osmosis membrane having a low NaCℓ inhibition rate and then contacting the thus obtained desalted solution with an aqueous solution containing methanol and hydrochloric acid to crystallize the α-APM hydrochloride thereby to effectively recover α-APM from the aforesaid solution and, further lies in specifying the amounts of α-A(M)PM and the inorganic chloride co-present thereby to markedly improve the recovery rate of α-APM and at the same time, to make devices required for recovery extremely simple and miniature.

Hereafter the present invention will be described in detail.

With respect to a method for effectively removing inorganic chlorides from a solution containing α-APM, α-A(M)P, α-AP, α-A(M)PM and inorganic chlorides, the present inventors have first made detailed investigations on the method for selectively removing inorganic chlorides alone using various reverse osmosis membranes having adjusted the NaCℓ inhibition rate to a low level without any loss of valuable components, paying their attention to reverse osmosis membranes which have come to be widely used recently from an aspect of economic advantages in turning salt water into fresh water or concentrating a solution containing valuable matters. Further with respect to a method for recovering α-APM from the desalted solution in a high yield, they have also extensively investigated a method capable of efficiently recovering α-APM from the solution in the highest yield throughout the desalting operation and the α-APM recovering operation.

As a result, it has been found that regarding the desalting operation, if the inhibition rate of NaCl is as high as 80% or more, water as a solvent is also permeated into the permeated liquid together with permeation of the inorganic chlorides and the absolute amount of the inorganic chlorides decreases if a long time is taken. However, the amount of the concentrate is much reduced and due to the increased concentration of the inorganic chlorides in the non-permeated liquid, the osmosis increases and becomes larger than the physical strength of the membrane to render the operation impossible and further, the permeation rate of the inorganic chlorides markedly decreases due to the increased salt concentration, etc. so that it is difficult to perform the procedures with a reverse osmosis membrane having a high NaCℓ inhibition rate in an industrial scale. Thus, it has been made clear that by choosing a reverse osmosis membrane whose NaCl inhibition rate is adjusted to not greater than 80%, the inorganic chloride co-present can be efficiently removed from the solution.

Furthermore, detailed investigations on the permeability of valuable compounds such as α-A(M)PM, α-APM, α-A(M)P, α-AP, etc. has revealed that the permeability of these valuable compounds is low and with a reverse osmosis membrane having the NaCℓ inhibition rate of approximately 30% or more, it is difficult for these compounds to permeate into the permeated liquid at the time of the desalting step, but the permeability of these valuable compounds suddenly increases with a reverse osmosis membrane showing the NaCℓ inhibition rate of approximately 30% or less. It has thus been made clear that for the purpose of the present invention, it is effective to use a reverse osmosis membrane having the NaCℓ inhibition rate of 30 to 80%.

The present inventors have also investigated the permeability of each of these valuable compounds in detail and as a result, they have unexpectedly found that α-A(M)PM has a specifically high permeability of the reverse osmosis membrane notwithstanding that α-A(M)PM has a larger molecular weight than other α-APM, α-A(M)P and α-AP. Namely, in case that the ratio of the α-A(M)PM content is relatively large in the desalting method of the present invention, it is disadvantageous that the amount of α-A(M)PM, which is one of the valuable compounds and permeated into the permeation liquid accompanied by desalting increases and as the result, the recovery rate of α-APM from these valuable compounds decreases. It has been found that in such a case, it is effective to demethyl-esterify α-A(M)PM co-present by means of heat treatment in a diluted hydrochloric acid aqueous solution under mild conditions, etc. to convert most of α-A(M)PM into α-APM, α-A(M)P or α-AP having low permeability and then subjecting to the desalting according to the present invention, thus preventing loss of the valuable compounds.

The reverse osmosis membranes which can be used in the desalting of the present invention include membranes of polysulfone type, polyamide type or cellulose acetate type, the NaCℓ inhibition rate of which is adjusted to approximately 30 to 80%, but the materials of the membranes are not restricted. The NaCℓ inhibition rate as used herein is a numerical value generally used to express permeability of a solute and is calculated from the concentration of NaCl in the permeated liquid when a 0.2% NaCℓ solution is supplied to a permeation device having mounted thereto a reverse osmosis membrane under a pressure of 30 bars G (kg/cm²)G at a temperature of 20°C, according to the following equation:

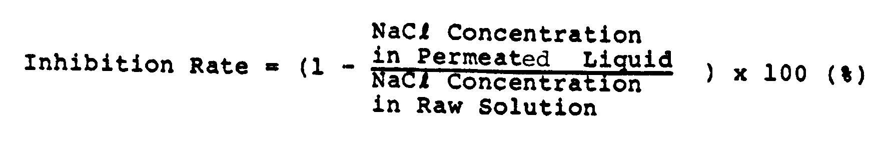

The temperature and pressure in the desalting operation may somewhat vary depending upon materials of the membrane used but in ordinary case, appropriate operational conditions can be chosen depending upon properties of the membrane, in the temperature range of 5 to 50°C and in the pressure range of 10 to 70 kg/cm²G (10 to 70 bars above atmospheric pressure).

The pH of the solution provided for the method of the present invention should be limited to a pH range corresponding to pH durability because the pH durability of the membrane varies basically depending upon materials of the membrane and operational temperature. Further the permeabilities of the inorganic chlorides, α-A(M)PM, etc. change more or less depending upon pH and therefore, it is desired to experimentally determine the pH range corresponding to the ratios of the components of the solution . In general, the pH ranges from 2 to 8 and is determined taking the foregoing into account.

Next, the present inventors have made various investigations on a method for recovering α-APM from the thus obtained desalted solution. It has thus been found that in order to recover α-APM in a high yield from an impure solution containing, in addition to α-APM, α-A(M)PM, α-A(M)P or α-AP, the most advantageous method comprises contacting the impure solution with an aqueous solution containing methanol and hydrochloric acid to once crystallize the α-APM hydrochloride, then subjecting the hydrochloride to solid-liquid separation and recover α-APM as α-APM hydrochloride crystals (U.S. Patent 3,798,207).

As a result of further detailed investigations on this crystallization step, it has been found that the recovery rate of α-APM decreases to cause disadvantages as the degree of the α-A(M)PM content in the desalted solution becomes high. Therefore, the present inventors have made investigations on this fact in further detail and as a result, they have made clear that upon crystallization of the α-APM hydrochloride by contacting with the aqueous solution containing methanol and hydrochloric acid, a long time is required for the conversion into α-APM when α-A(M)PM is present in large quantities because the conversion rate of α-A(M)PM into α-APM in the solution is slow as compared to the rate of conversion of other compounds, i.e. α-A(M)P and α-AP into α-APM. Therefore, when the crystallization is performed in an industrial scale, most of α-A(M)PM is not converted into α-APM but remains in the solution thereby to decrease the recovery rate of α-APM.

In view of the foregoing facts, one of the features of the present invention is that by specifying the ratio of the α-A(M)PM content in the solution provided for the recovery method according to the present invention, the loss of α-A(M)PM upon desalting is avoided and at the same time, the solution is brought into contact with the aqueous solution containing methanol and hydrochloric acid to recover α-APM as the α-APM hydrochloride, whereby the efficiency is markedly improved to miniaturize the crystallization device and make great reduction in device costs possible.

The present inventors have further made careful investigations on the amount of the inorganic chlorides which affect the recovery rate in the recovery of α-APM as the α-APM hydrochloride and have made clear that if the ratio of the inorganic chloride content to the valuable compounds such as α-APM, etc. is approximately 0.3 to 1.0 by weight; even though desalting is not performed as excessively as 0.3 or less, the recovery rate of α-APM can be maintained in a high rate. If the ratio is 1.0 or more, the inorganic chlorides are precipitated upon the crystallization of the α-APM hydrochloride and in order to avoid such precipitation, water or the like should be added from an external source to dissolve the inorganic chlorides and, in such a case, the concentrations of α-APM and its derivatives are relatively decreased so that the crystallization rate of the α-APM hydrochloride decreases and the recovery rate of α-APM decreases. Another feature of the present invention lies in that the solution provided for the recovery method of the present invention, in which the ratio of the inorganic chloride content to the total sum of the valuable matters such as α-APM and the like is not less than 1.0 in a weight ratio, is desalted up to 0.3 to 1.0 in a weight ratio, using the reverse osmosis membrane having such a property that the NaCℓ inhibition rate is 30 to 80%, avoiding increase in desalting load due to excessive desalting which results in a reduction of the equipment costs and operation costs because of cutback in the desalting installation.

It is desired that the amount of methanol used in the present invention be experimentally determined in a range of about 2 mols or less per 1 liter of the desalted solution, since the suitable amount varies depending upon the ratios of α-A(M)PM, α-APM, α-A(M)P and α-AP contained in the desalted solution. When the amount of methanol exceeds about 2 mols per 1 liter of the desalted solution, methyl-esterification of α-APM is accelerated to increase the formation of α-A(M)PM so that the recovery rate of α-APM is reduced; further also when the amount of methanol is as low as less than 1 mol per 1 mole of α-AP, the recovery rate of α-APM is reduced.

It is necessary that the amount of hydrochloric acid be appropriately determined in such a range that the inorganic chlorides co-present in the desalted solution are not precipitated; it is generally preferred that the hydrochloric acid be present in the range of about 0.5 to about 5 mols per 1 liter of the desalted solution and in an amount at least equimolar to the total mol number of α-A(M)PM, α-APM, α-A(M)P and α-AP, from a standpoint of increasing the yield.

Other solvents that do not inhibit the addition of α-APM to hydrochloric acid, for example, ethylene glycol, acetone, etc. may also be incorporated in such an amount that does not markedly reduce the solubility of the inorganic chlorides, upon crystallization of the α-APM hydrochloride by contacting with the aqueous solution containing methanol and hydrochloric acid. In any event,the examination and determination of optimum conditions within the given conditions can be easily made to one skilled in the art.

Hereafter the present invention will be described in detail with reference to the examples below.

### Example 1

A raw solution having a pH of 4.54, containing 8.7 g of α-A(M)PM, 5.3 g of α-APM, 2.9 g of α-AP and 27.3 g of NaCℓ per 1 liter of the solution was supplied to a permeation device having mounted thereto spiral polyamide reverse osmosis membranes having NaCℓ inhibition rates of 80, 60, 30% and 15% (control), respectively, at a temperature of 20°C under a pressure of 40 kg/cm²G. Desalting and concentration were performed, while cyclizing and mixing a non-permeation solution to and with the raw solution.

From the composition of the permeation liquid exhausted in this case, the inhibition rate of each component was calculated according to the following equation.

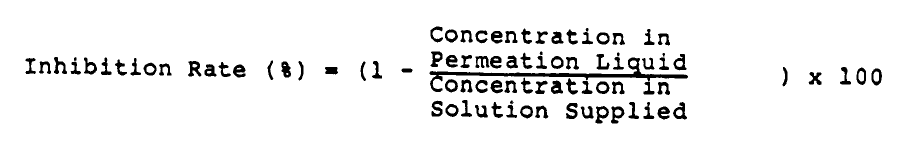

The results are shown in Table 1. Analysis method: liquid chromatography (α-A(M)PM, α-APM, α-AP), (Cℓ⁻).

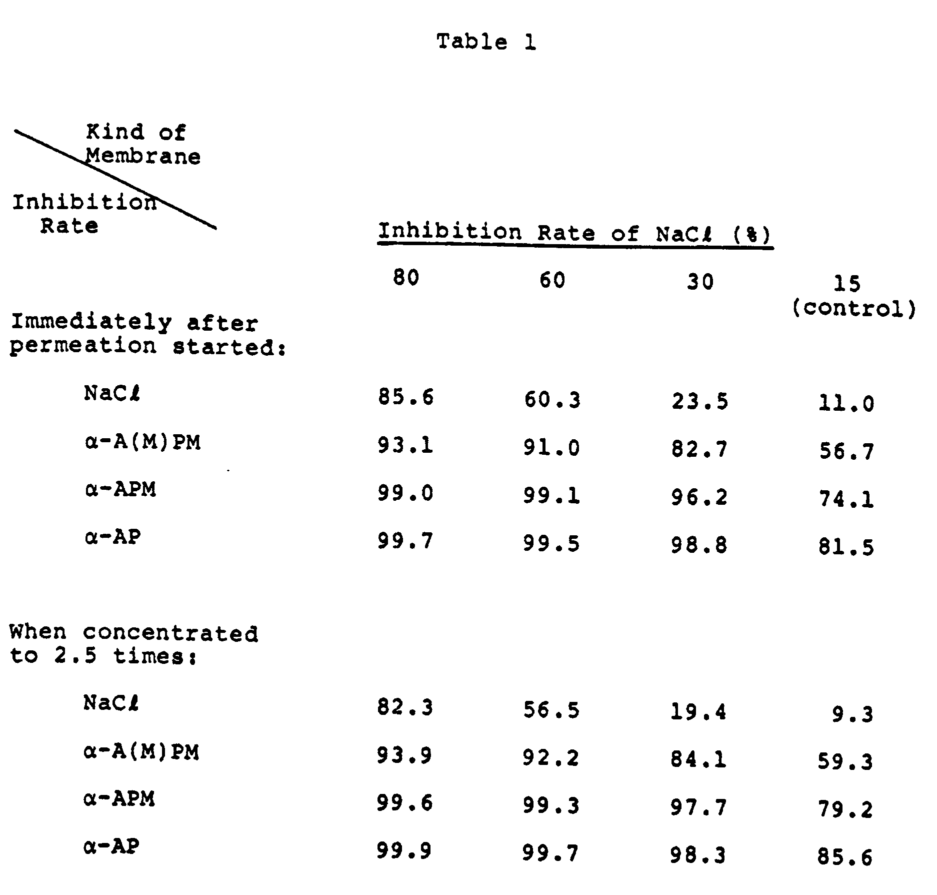

From this example, it will be understood that when the NaCℓ inhibition rate of the reverse osmosis membrane is lower than 30%, the recovery rate of the valuable matters is seriously reduced. At the same time, it is also evident that α-A(M)PM in the valuable matters has a specifically readily permeable property notwithstanding that its molecular weight is large.

### Example 2

While cooling, 28% NH₃ water was added to the mother liquor obtained by separating α-APM hydrochloride, which contained 0.89% of α-APM, 1.54% of α-A(M)PM, 0.31% of α-A(M)P, 0.48% of α-AP and 12.6% of HCℓ to neutralize to pH of 5.2. The neutralized solution was supplied to flat membrane type permeation devices having mounted thereto polyamide type reverse osmosis membrane (A) having the NaCℓ inhibition rate of 50% and for comparison, polysulfone type reverse osmosis membrane having the NaCℓ inhibition rate of 25%, respectively, at a temperature of 25°C under a pressure of 40 kg/cm²G. While cyclizing a non-permeation liquid to a neutralizing liquid tank for supplying to the permeation device, the solution was desalted and concentrated until the volume of the neutralizing liquid became the original volume. Next, water was added until the volume became the original volume. Further by repeating similar operation, the system was desalted and concentrated until the volume became the half of the original volume. The results are shown in Table 2.

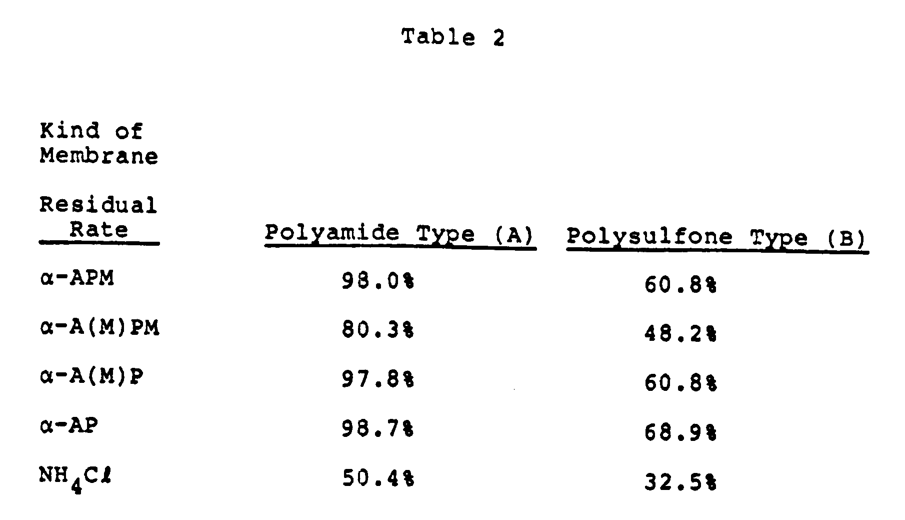

From this example, it is understood and evident that the results are the same as in Example 1.

### Example 3

Under reduced pressure, 1.5 liters of a solution containing 9.2 g of α-APM, 26.8 g of α-A(M)PM, 7.6 g of α-AP, 2.2 g of α-A(M)P and 38 g of NaCℓ per 1 liter of the solution until the volume was reduced to 0.6 liters. The concentrate was divided into 3 aliquots (A, B and C), each of which was charged in a 3-necked flask of 500 ml equipped with a stirrer. Methanol, 8 ml, and 43 ml of 35% hydrochloric acid were added to A and 10 ml of 35% hydrochloric acid was added to B. Each mixture was heated at 40°C for 2 hours to demethyl-esterify approximately 20% of α-A(M)PM. Then, 8 ml of methanol and 33 ml of 35% hydrochloric acid were added. To C was added 20 ml of 35% hydrochloric acid and the mixture was heated at 40°C for 4 hours to demethyl-esterify approximately 75% of α-A(M)PM. Then, 8 ml of methanol and 23 ml of 35% hydrochloric acid were added thereto. Next, each mixture was put in a thermostat adjusted to 10°C to perform crystallization for 3 days with stirring. The precipitated crystals were taken by filtration. After washing with a small amount of cold 2 N hydrochloric acid, the crystals were dried at 50°C under reduced pressure.

Infrared absorption spectra of these crystals were identical with those of α-APM hydrochloride. The results on yield, recovery rate and purity (liquid chromatography) are shown in Table 3.

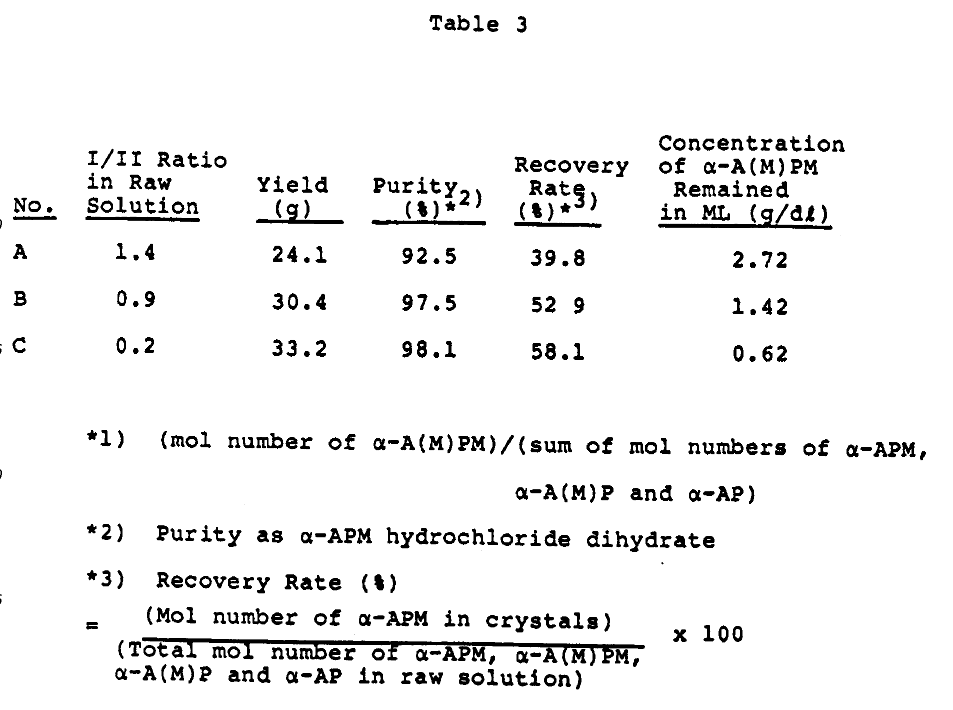

From this example, it will be easily understood that when the I/II ratio in the raw solution provided for the crystallization step is greater than 1.0, the yield of α-APM as the product is markedly reduced.

### Example 4

α-APM was crystallized by neutralizing impure α-APM hydrochloride containing 2.2% of α-AP and 3.4% of α-A(M)PM. The thus obtained mother liquor was further concentrated and again subjected to crystallization of α-APM. The mother liquor (composition: 0.95 g/dℓ of α-APM, 0.77 g/dℓ of α-AP, 0.97 g/dℓ of α-A(M)PM, 0.22 g/dℓ of α-A(M)P, 0.17 g/dℓ of DKP and 5.9 g/dℓ of NH₄Cℓ, pH 4.9) obtained by separating α-APM underwent repeated concentration and dialysis operation in a manner as described in Example 2, using a polyamide type reverse osmosis membrane having the NaCℓ inhibition rate of 50%. Thus, 4 kinds of the treated solutions having various desalting rates of NH₄Cℓ were obtained.

The results are shown in Table 4.

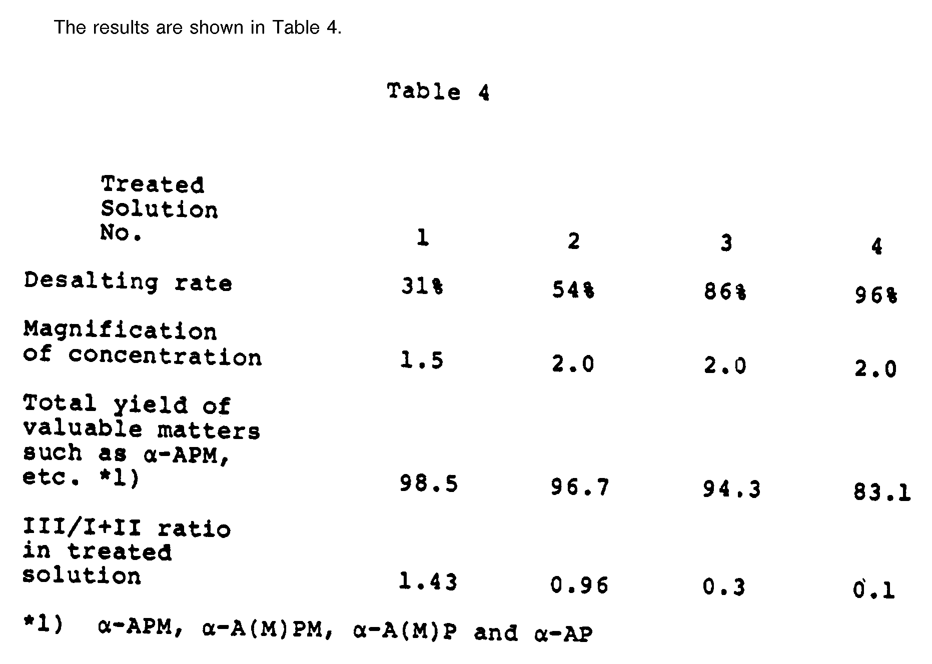

The 4 desalted solutions obtained by the procedure described above were concentrated to 1.65 g/dℓ in the concentration of the valuable matters such as α-APM, etc., calculated as the total nitrogen. Each concentrate was charged in a 3-necked flask of 300 ml equipped with a stirrer. Methanol, 6 ml, and 51 ml of 35% hydrochloric acid were added to each concentrate and each mixture was put in a thermostat adjusted to 10°C to perform crystallization for 3 days with stirring. The precipitated crystals were taken by filtration. After washing with a small amount of cold 2 N hydrochloric acid, the crystals were dried at 50°C under reduced pressure.

Infrared absorption spectra of these crystals were identical with those of α-APM hydrochloride. The results on yield, recovery rate and purity (liquid chromatography) are shown in Table 5.

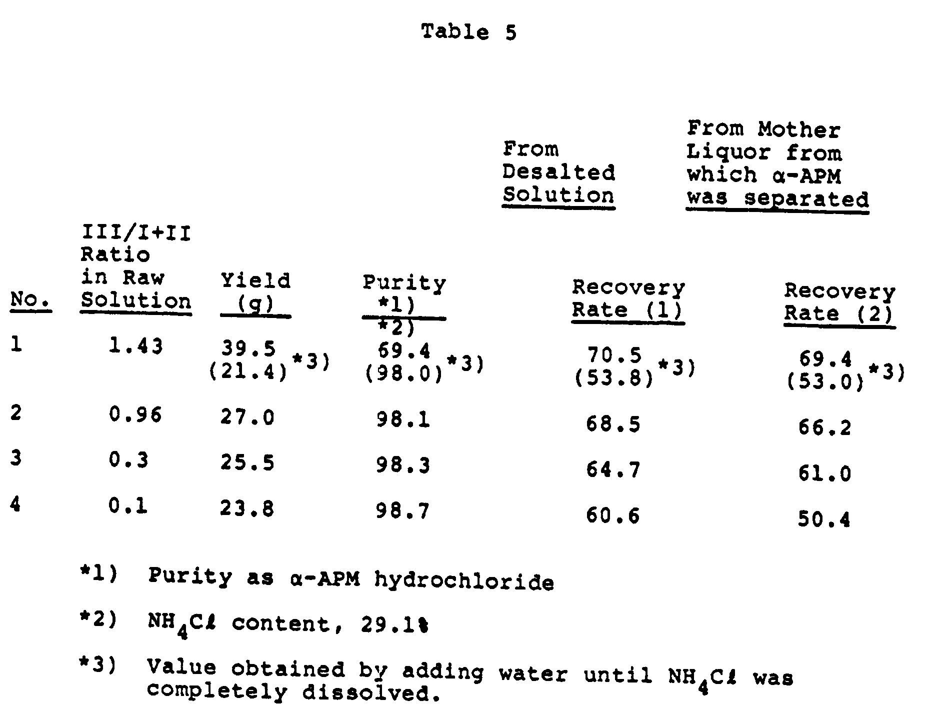

From this example, it will be understood that when the III/I+II ratio in the treated solution is greater than 1.0, the obtained crystals are not pure and labors are required for re-purification and for this reason, the yield is markedly reduced. On the other hand, it will be understood that also when the III/I+II ratio is lower than 0.3, the valuable matters are consequentially shifted to the permeation liquid together with the salts to cause loss of the valuable matters so that decrease in the yield results.

## Claims

1. A process for recovering α-L-aspartyl-L-phenylalanine-methyl ester (α-APM) or the hydrochloride thereof from a solution mixture containing (I) α-L-aspartyl-L-phenylalanine dimethyl ester (α-A(M)PM), and the corresponding demethylation derivatives (II), i.e. α-L-aspartyl-L-phenylalanine methyl ester (α-APM), α-L-aspartyl methyl ester-L-phenylalanine, wherein the methyl ester group is bound to the β-carboxy group of the aspartyl residue (α-A(M)P),and α-L-aspartyl-L-phenylalanine (α-AP), and at least one inorganic chloride, comprising desalting and concentrating a solution mixture in which the weight ratio of (I) to (II) is not greater than 1.0 and the ratio of the inorganic chloride content (III) to the sum of the compounds (I+II), i.e. (III)/(I+II) in terms of the weight ratio is not less than 1.0, by means of a reverse osmosis membrane having a NaCl inhibition rate of 30 to 80 % until said ratio of the inorganic chloride content to the content of compounds (I) and (II), i.e. (III)/(I+II) attains a value in the range of 0.3 to 1.3 in terms of the weight ratio,
then bringing the concentrate into contact with an aqueous solvent containing methanol and hydrochloric acid to precipitate α-L-aspartyl-L-phenylalanine methyl ester (α-APM) hydrochloride and optionally neutralizing the hydrochloride.

2. A process according to claim 1, wherein the ratio (I)/(II) is adjusted to a value in the stated range by saponification of α-A(M)PM under mild conditions to convert most of α-A(M)PM into α-APM, α-A(M)P and/or α-AP.

3. A process according to either of the claims 1 or 2, wherein the reverse osmosis is carried out at a temperature in the range of 5 to 50°C and an elevated pressure of 10 to 70 bars.

4. A process according to any of the claims 1 to 3, wherein the amount of methanol is adjusted to a value in the range of about 2 moles or less per 1 l of the desalted solution.

5. A process according to any of the claims 1 to 4, wherein the amount of hydrochloric acid is in the range of about 0.5 to about 5 moles per 1 l of the desalted solution.

## Patentansprüche

1. Verfahren zur Gewinnung von α-L-Aspartyl-L-phenylalaninmethylester (α-APM) oder des Hydrochlorids desselben aus einer Lösungsmischung, welche (I) α-L-Aspartyl-L-phenylalanindimethylester (α-A(M)PM), und die entsprechenden demethylierten Derivate (II), d.h. α-L-Aspartyl-L-phenylalaninmethylester (α-APM), α-L-Aspartylmethylester-L-phenylalanin, worin die Methylestergruppe an die β-Carboxygruppe des Aspartylrestes gebunden ist (α-A(M)P), und α-L-Aspartyl-L-phenylalanin (α-AP), und mindestens ein anorganisches Chlorid enthält, das umfaßt :
Entsalzen und Konzentrieren einer Lösungsmischung, in welcher das Gewichtsverhältnis von (I):(II) nicht größer als 1,0 und das Verhältnis des Anteils des anorganischen Chlorids (III) zu der Summe der Verbindungen (I+II), d.h. (III)/(I+II) ausgedrückt als Gewichtsverhältnis, nicht geringer als 1,0 ist, mittels einer Umkehrosmose-Membran mit einer NaCl Abweisungsrate von 30 bis 80 %, bis das Verhältnis des Anteils des anorganischen Chlorids zum Anteil der Verbindungen(I) und (II), d.h. (III)/(I+II) einen Wert im Bereich von 0,3 bis 1,3, ausgedrückt als Gewichtsverhältnis,
und anschließendes Inberührungbringen des Konzentrats mit einem Methanol und Salzsäure enthaltenden wässerigen Lösungsmittel, um α-L-Aspartyl-L-phenylalaninmethylester (α-APM) als Hydrochlorid auszufällen und
gegebenenfalls Neutralisieren des Hydrochlorids.

2. Verfahren gemäß Anspruch 1, bei dem das Verhältnis (I)/(II) durch Verseifung des α-A(M)PM unter milden Bedingungen um die überwiegende Menge des α-A(M)PM in α-APM, α-A(M)P und/oder α-AP umzuwandeln, auf einen Wert im angegebenen Bereich eingestellt wird.

3. Verfahren gemäß einem der Ansprüche 1 oder 2, bei dem die Umkehrosmose bei einer Temperatur im Bereich von 5 bis 50°C und einem erhöhten Druck von 10 bis 70 bar ausgeführt wird.

4. Verfahren gemäß einem der Ansprüche 1 bis 3, bei dem die Menge des Methanols auf einen Wert im Bereich von ungefähr 2 Mol oder weniger pro 1 Liter der entsalzten Lösung eingestellt wird.

5. Verfahren gemäß einem der Ansprüche 1 bis 4, bei dem die Menge der Chlorwasserstoffsäure im Bereich von ungefähr 0,5 bis ungefähr 5 Mol pro 1 Liter der entsalzten Lösung liegt.

## Revendications

1. Procédé pour récupérer l'ester méthylique d'α-L-aspartyl-L-phénylalanine (α-APM) ou son chlorhydrate à partir d'un mélange en solution contenant (I) l'ester diméthylique d'α-L-aspartyl-L-phénylalanine (α-A(M)PM) et les dérivés de déméthylation correspondants (II), c'est-à-dire l'ester méthylique d'α-L-aspartyl-L-phénylalanine, l'α-L-β-méthyl-aspartyl-L-phénylalanine dans laquelle le groupe ester méthylique est lié au groupe β-carboxy du reste aspartyle (α-A(M)P) et l'α-L-aspartyl-L-phénylalanine (α-AP) et au moins un chlorure inorganique, comprenant l'élimination des sels et la concentration d'un mélange en solution dans lequel le rapport pondéral de (I) à (II) est de pas plus de 1,0 et le rapport de la teneur en chlorures inorganiques (III) à la somme des composés (I + II), c'est-à-dire (III)/(I+II), est de pas moins de 1,0 en poids, au moyen d'une membrane d'osmose inverse ayant un taux d'inhibition de NaCl de 30 à 80% jusqu'à ce que ledit rapport de la teneur en chlorures inorganiques à la teneur en composés (I) et (II), c'est-à-dire (III)/(I+II), atteigne une valeur dans la gamme de 0,3 à 1,3 en poids,
ensuite la mise en contact du concentré avec un solvant aqueux contenant du méthanol et de l'acide chlorhydrique pour précipiter le chlorhydrate d'ester méthylique d'α-L-aspartyl-L-phénylalanine (α-APM) et facultativement la neutralisation du chlorhydrate.

2. Procédé selon la revendication 1, dans lequel le rapport (I)/(II) est réglé à une valeur dans la gamme indiquée par saponification de l'α-A(M)PM en conditions douces pour convertir la majeure partie de l'α-A(M)PM en α-APM, α-A(M)P et/ou α-AP.

3. Procédé selon la revendication 1 ou 2, dans lequel l'osmose inverse est mise en oeuvre à une température dans la gamme de 5 à 50°C et sous une pression élevée de 10 à 70 bar.

4. Procédé selon l'une quelconque des revendications 1 à 3, dans lequel la quantité de méthanol est réglée à une valeur dans la gamme d'environ 2 mol ou moins par litre de la solution débarrassée des sels.

5. Procédé selon l'une quelconque des revendications 1 à 4, dans lequel la quantité d'acide chlorhydrique est dans la gamme d'environ 0,5 à environ 5 mol par litre de la solution débarrassée des sels.
